# EUROPEAN PATENT APPLICATION

(11) **EP 4 344 602 A1**
(43) Date of publication of application: **03.04.2024**
(21) Application number: 22903354.3
(22) Date of filing: 05.12.2022
(51) Int. Cl.: A61B 1/00, A61B 1/005, F16L 21/025

(54) **TIP AND CONTINUUM BODY CONNECTING STRUCTURE AND ENDOSCOPE**

(30) Priority: 07.12.2021 CN 202123054694 U
(71) Applicant: Guangzhou Red Pine Medical Instrument Co., Ltd., Guangzhou, Guangdong 510000 (CN)
(72) Inventor: YI, Feng, Guangzhou, Guangdong 510000 (CN); LI, Jing, Guangzhou, Guangdong 510000 (CN); TAN, Xiaofeng, Guangzhou, Guangdong 510000 (CN)
(74) Representative: Murgitroyd & Company
(86) International application number: PCT/CN2022/136465
(87) International publication number: WO 2023/103932

(57) **Abstract**

A tip and continuum body connecting structure (100) and an endoscope. The tip and continuum body connecting structure (100) comprises: a tip (110), which is provided with first snap-fit portions (111), the first snap-fit portions (111) being located in the circumferential direction of the tip (110); and a continuum body (120), which is sleeved with the tip (110), the continuum body (120) being provided with second snap-fit portions (115), the second snap-fit portions (115) being located in the circumferential direction of the continuum body (120), and the first snap-fit portions (111) being snap-fitted with the second snap-fit portions (115), such that the tip (110) is connected to the continuum body (120). In the present tip and continuum body connecting structure (100) and endoscope, the snap-fit connection facilitates installation, is suitable for the connection of a continuum body (120) and tip (110) made of plastic material, and is beneficial for improving the operating convenience of the connection between the continuum body (120) and the tip (110). In addition, the first snap-fit portions (111) are provided in the circumferential direction of the tip (110) and the second snap-fit portions (115) are provided in the circumferential direction of the continuum body (120), and the connection of the first snap-fit portions and the second snap-fit portions can improve the stability of the connection of the tip (110) and the continuum body (120), thereby preventing the tip (110) from falling off the continuum body (120), improving the reliability of the endoscope, and reducing the probability of medical accidents.

## Description

### TECHNICAL FIELD

The present application relates to the technical field of medical devices, and in particular, to a connecting structure for a distal tip and a bending section, and an endoscope.

### BACKGROUND

With the development of a medical technology, an endoscope technology has emerged. An endoscope is configured to visually inspect hard-to-reach sites such as organs in a human body to observe lesions. Generally, the endoscope includes a long insertion tube. The long insertion tube is provided with a handle at an end thereof proximal to an operator and a visual inspection device such as a built-in camera at a distal end thereof. In order to control the endoscope inside the human body, the endoscope includes a bendable bending section. An orientation of a distal tip on an end portion of the bending section is adjusted by bending the bending section, thereby changing a viewing angle.

In the conventional art, in order to adjust the orientation of the distal tip of the endoscope, there is a need to connect the distal tip made of plastic materials to the bending section made of plastic materials. In the conventional endoscope, the distal tip is connected to the bending section mainly by bonding. However, since the material used for the bending section has extremely poor bonding properties, the bonding manner easily causes the distal tip to fall off the bending section, resulting in poor reliability and leading to medical accidents.

### SUMMARY

Accordingly, it is necessary to provide a connecting structure for a distal tip and a bending section, and an endoscope, which can effectively improve the stability of the connection between the distal tip and the bending section and reduce the probability of medical accidents.

A connecting structure for a distal tip and a bending section includes: a distal tip provided with first engaging portions, the first engaging portions being located in a circumferential direction of the distal tip; and a bending section coupled to the distal tip, the bending section being provided with second engaging portions, the second engaging portions being located in a circumferential direction of the bending section, and the first engaging portions being snap-fitted with the second engaging portions, such that the distal tip is connected to the bending section.

In the above connecting structure for a distal tip and a bending section, during mounting, the first engaging portions on the distal tip are snap-fitted with the second engaging portions on the bending section, such that the distal tip is connected to the bending section. The engaging connection facilitates mounting, is suitable for the connection between the bending section made of plastic materials and the distal tip made of plastic materials, and is beneficial for improving the operating convenience of the connection between the bending section and the distal tip. In addition, the first engaging portions are provided in the circumferential direction of the distal tip, and the second engaging portions are provided in the circumferential direction of the bending section. The connection between the first engaging portions and the second engaging portions can improve the stability of the connection between the distal tip and the bending section, thereby preventing the distal tip from falling off the bending section, improving the reliability of the endoscope, and reducing the probability of medical accidents.

In an embodiment, at least two first engaging portions and at least two second engaging portions are provided. The at least two first engaging portions are arranged at intervals in the circumferential direction of the distal tip. The at least two second engaging portions are arranged at intervals in the circumferential direction of the bending section. The at least two first engaging portions and the at least two second engaging portions are arranged in one-to-one correspondence.

In an embodiment, the first engaging portions are formed as engaging posts, the second engaging portions are formed as engaging holes. Cross-sectional profiles of the engaging posts are adapted to opening shapes of the engaging holes respectively.

In an embodiment, the first engaging portions are formed as engaging holes, the second engaging portions are formed as engaging posts. Opening shapes of the engaging holes are adapted to cross-sectional profiles of the engaging posts respectively.

In an embodiment, the engaging posts are provided with introducing inclined surfaces. The introducing inclined surfaces is configured to guide the engaging posts into the engaging holes respectively.

In an embodiment, the at least two engaging posts include at least a first engaging post and a second engaging post. The at least two engaging holes include at least a first engaging hole and a second engaging hole. The first engaging post is snap-fitted with the first engaging hole, and the second engaging post is snap-fitted with the second engaging hole. A cross-sectional profile of the first engaging post is different from that of the second engaging post. An opening shape of the first engaging hole is different from that of the second engaging hole.

In an embodiment, the cross-sectional profile of the first engaging post is in a waist shape extending in an axial direction of the distal tip, and the opening shape of the first engaging hole is in a waist shape extending in an axial direction of the bending section. The cross-sectional profile of the second engaging post is circular, and the opening shape of the second engaging hole is circular.

In an embodiment, the distal tip is provided with a first annular connecting portion. The first annular connecting portion is connected to the distal tip and extending in an axial direction of the distal tip. The bending section is provided with a second annular connecting portion. The second annular connecting portion is connected to the bending section and extending in an axial direction of the bending section. The first engaging portions are located in a circumferential direction of the first annular connecting portion. The second engaging portions are located in a circumferential direction of the second annular connecting portion. An outer diameter of the first annular connecting portion is less than an inner diameter of the second annular connecting portion. The second annular connecting portion is sleeved outside the first annular connecting portion. The distal tip is engaged with the second annular connecting portion. An outer wall of the distal tip smoothly transitions to an outer wall of the second annular connecting portion.

In an embodiment, a sealant is applied between an outer wall of the first annular connecting portion and an inner wall of the second annular connecting portion.

In an embodiment, the distal tip is provided with a first annular connecting portion. The first annular connecting portion is connected to the distal tip and extends in an axial direction of the distal tip. The bending section is provided with a second annular connecting portion. The second annular connecting portion is connected to the bending section and extends in an axial direction of the bending section. The first engaging portions are located in a circumferential direction of the first annular connecting portion. The second engaging portions are located in a circumferential direction of the second annular connecting portion. An outer diameter of the second annular connecting portion is less than an inner diameter of the first annular connecting portion. The first annular connecting portion is sleeved outside the second annular connecting portion. The bending section is engaged with the first annular connecting portion. An outer wall of the bending section smoothly transitions to an outer wall of the first annular connecting portion.

In an embodiment, a sealant is applied between an outer wall of the second annular connecting portion and an inner wall of the first annular connecting portion.

An endoscope includes the connecting structure for a distal tip and a bending section according to any one of the above embodiments.

In the above endoscope, during mounting, the first engaging portions on the distal tip are snap-fitted with the second engaging portions on the bending section, such that the distal tip is connected to the bending section. The snap-fit connection facilitates mounting, is suitable for the connection between the bending section made of plastic materials and the distal tip made of plastic materials, and is beneficial for improving the operating convenience of the connection between the bending section and the distal tip. In addition, the first engaging portions are provided in the circumferential direction of the distal tip, and the second engaging portions are provided in the circumferential direction of the bending section, and the connection between the first engaging portions and the second engaging portions can improve the stability of the connection between the distal tip and the bending section, thereby preventing the distal tip from falling off the bending section, improving the reliability of the endoscope, and reducing the probability of medical accidents.

### BRIEF DESCRIPTION OF THE DRAWINGS

The accompanying drawings forming part of the present application are intended to provide further understanding of the present application. Exemplary embodiments of the present application and descriptions thereof are intended to explain the present application, and do not constitute any inappropriate limitation on the present application.

In order to more clearly illustrate the technical solutions in embodiments of the present application, the accompanying drawings used in the description of the embodiments will be briefly introduced below. It is apparent that, the accompanying drawings in the following description are only some embodiments of the present application, and other drawings can also be obtained by those of ordinary skill in the art from the provided drawings without creative efforts.
FIG. 1 is a schematic structural view of a connecting structure for a distal tip and a bending section according to an embodiment.
FIG. 2 is a schematic view of a combined structure of the connecting structure for a distal tip and a bending section according to an embodiment.
FIG. 3 is a schematic enlarged view of a structure at portion A in FIG. 2.

### Illustrations for reference signs:

100: connecting structure for a distal tip and a bending section; 110: distal tip; 111: first engaging portion; 112: engaging post; 113: introducing inclined surface; 114: first engaging post; 115: second engaging post; 116: first annular connecting portion; 120: bending section; 121: second engaging portion; 122: engaging hole; 123: first engaging hole; 124: second engaging hole; 125: second annular connecting portion.

### DETAILED DESCRIPTION OF THE EMBODIMENTS

In order to make the above objects, features, and advantages of the present application more obvious and understandable, specific implementations of the present application are described in detail below with reference to the accompanying drawings. In the following description, many specific details are set forth in order to fully understand the present application. However, the present application can be implemented in many other ways different from those described herein, and those skilled in the art can make similar improvements without departing from the connotation of the present application. Therefore, the present application is not limited by the specific embodiments disclosed below.

In the description of the present application, it should be understood that the orientation or position relationships indicated by the terms "central", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", and the like are based on the orientation or position relationships shown in the accompanying drawings and are intended to facilitate the description of the present application and simplify the description only, rather than indicating or implying that the device or element referred to must have a particular orientation or be constructed and operated in a particular orientation, and therefore are not to be interpreted as limiting the present application.

In addition, the terms "first" and "second" are used for descriptive purposes only, which cannot be construed as indicating or implying a relative importance, or implicitly specifying the number of the indicated technical features. Therefore, the features defined by "first" and "second" may explicitly or implicitly include at least one of the features. In the description of the present application, "a plurality of' means two or more, such as two or three, unless specifically stated otherwise.

In the present application, unless otherwise specifically stated and defined, the terms such as "mounting", "coupling", "connection", and "fixation" should be understood in a broad sense, such as, a fixed connection, a detachable connection, or an integral connection; a mechanical connection, or an electrical connection; a direct connection, an indirect connection through an intermediate medium; an internal connection between two elements, or interaction between two elements, unless otherwise specifically defined. For those of ordinary skill in the art, the specific meanings of the foregoing terms in the present application can be understood on a case-by-case basis.

In the present application, unless otherwise explicitly specified and defined, the expression a first feature being "on" or "under" a second feature may be the case that the first feature is in direct contact with the second feature, or the first feature is in indirect contact with the second feature via an intermediate medium. Furthermore, the expression a first feature being ""over", "above", or "on top of" a second feature may be the case that the first feature is directly above or obliquely above the second feature, or only means that the level of the first feature is higher than that of the second feature. The expression a first feature being "below", "underneath", or "under" a second feature may be the case that the first feature is directly underneath or obliquely underneath the second feature, or only means that the level of the first feature is lower than that of the second feature.

It should be noted that when one element is referred to as "fixed to" or "arranged on" another element, it may be directly disposed on the other element or an intermediate element may exist. When one element is considered to be "connected to" another element, it may be directly connected to the other element or an intermediate element may co-exist. The terms "perpendicular", "horizontal", "left", "right", and similar expressions used herein are for illustrative purposes only, and do not represent the unique implementation.

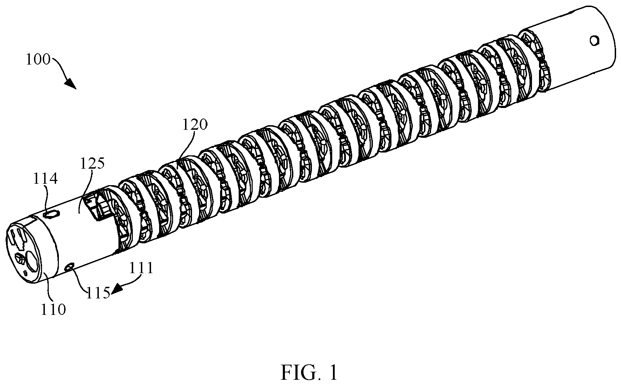

In an embodiment, referring to FIGS. 1, 2, and 3, a connecting structure for a distal tip and a bending section 100 includes a distal tip 110 and a bending section 120. The distal tip 110 is provided with first engaging portions 111. The first engaging portions 111 are located in the circumferential direction of the distal tip 110. The bending section 120 is coupled to the distal tip 110. The bending section 120 is provided with second engaging portions 121. The second engaging portions 121 are located in the circumferential direction of the bending section 120. The first engaging portions 111 are snap-fitted with the second engaging portions 121, such that the tip 110 is connected to the bending section 120.

In the above connecting structure for a distal tip and a bending section 100, during mounting, the first engaging portions 111 on the distal tip 110 are snap-fitted with the second engaging portions 121 on the bending section 120, such that the distal tip 110 is connected to the bending section 120. The snap-fit connection facilitates mounting, is suitable for the connection between the bending section 120 made of plastic materials and the distal tip 110made of plastic materials, and is beneficial for improving the operating convenience of the connection between the bending section 120 and the distal tip 110. In addition, the first engaging portions 111 are provided in the circumferential direction of the distal tip 110, and the second engaging portions 121 are provided in the circumferential direction of the bending section 120, such that the connection between the first engaging portions and the second engaging portions can improve the stability of the connection between the distal tip 110 and the bending section 120, thereby preventing the distal tip 110 from falling off the bending section 120, improving the reliability of the endoscope, and reducing the probability of medical accidents.

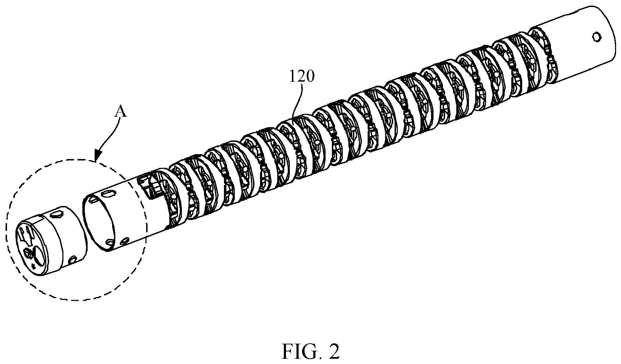

Further, referring to FIGS. 1 and 2, at least two first engaging portions 111 and at least two second engaging portions 121 are provided. The at least two first engaging portions 111 are arranged at intervals in the circumferential direction of the distal tip 110, and the at least two second engaging portions 121 are arranged at intervals in the circumferential direction of the bending section 120.

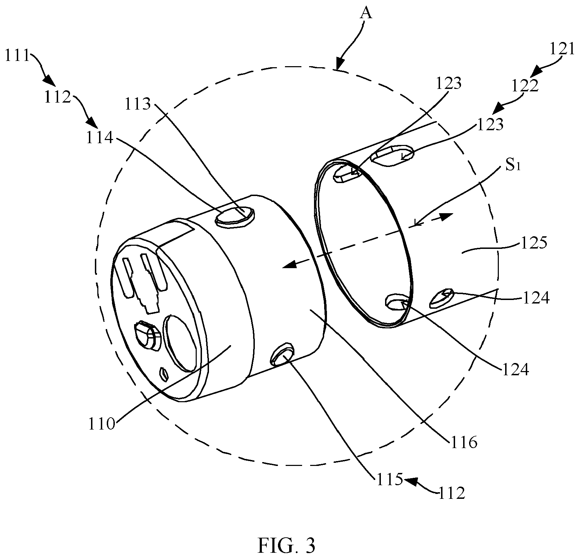

Further, referring to FIGS. 2 and 3, four first engaging portions 111 are provided. The four first engaging portions 111 are arranged at intervals in the circumferential direction of the distal tip 110. Four second engaging portions 121 are provided. The four second engaging portions 121 are arranged at intervals in the circumferential direction of the bending section 120. In this way, on the one hand, it is beneficial to improve the operating convenience of the snap-fit connection, and facilitates mounting of the distal tip 110. On the other hand, it is beneficial to disperse stress at the connection and improve stability of the circumferential connection between the bending section 120 and the distal tip 110, which prevents loosening and falling of the distal tip 110, thereby preventing occurrence of medical accidents during inspection of the endoscope. Although this embodiment provides only an option for quantities of the first engaging portions 111 and the second engaging portions 121, but which are not limited thereto.

Furthermore, referring to FIGS. 2 and 3, the at least two first engaging portions 111 and the at least two second engaging portions 121 are arranged in one-to-one correspondence. In this way, it is beneficial to further improve the stability of the circumferential connection between the bending section 120 and the distal tip 110.

In an embodiment, referring to FIGS. 2 and 3, the first engaging portions 111 are formed as engaging posts 112, the second engaging portions 121 are formed as engaging holes 122. Cross-sectional profiles of the engaging posts 112 are adapted to opening shapes of the engaging holes 122 respectively. In this way, through the connection between the engaging posts 112 and the engaging holes 122, it is beneficial to ensure stability of the snap-fit connection. In addition, the engaging posts 112 and the engaging holes 122 adapted to each other in shape help to prevent loosening, thereby improving overall quality of the endoscope.

Alternatively, in another embodiment (not shown), the first engaging portions 111 are formed as engaging holes 122, the second engaging portions 121 are formed as engaging posts 112. Opening shapes of the engaging holes 122 are adapted to cross-sectional profiles of the engaging posts 112 respectively. In this way, through the connection between the engaging posts 112 and the engaging holes 122, it is beneficial to ensure stability of the snap-fit connection. In addition, the engaging posts 112 and the engaging holes 122 adapted to each other in shape help to prevent loosening, thereby improving overall quality of the endoscope.

Further, referring to FIGS. 2 and 3, the engaging posts 112 are provided with introducing inclined surfaces 113 respectively. The introducing inclined surfaces 113 are configured to guide the engaging posts 112 into the engaging holes 122 respectively. In this way, it is convenient for the engaging posts 112 to enter the engaging holes 122 when the bending section 120 is connected to the distal tip 110, thereby improving the convenience of the snap-fit connection between the distal tip 110 and the bending section 120.

In an embodiment, referring to FIGS. 2 and 3, the at least two engaging posts 112 include at least a first engaging post 114 and a second engaging post 115, and the at least two engaging holes 122 include at least a first engaging hole 123 and a second engaging hole 124. The first engaging post 114 is snap-fitted with the first engaging hole 123, and the second engaging post 115 is snap-fitted with the second engaging hole 124. A cross-sectional profile of the first engaging post 114 is different from that of the second engaging post 115, and an opening shape of the first engaging hole 123 is different from that of the second engaging hole 124. In this way, the different shapes of the first engaging post 114 and the second engaging post 115 are used as distinctions, which facilitates a directional connection of the distal tip 110 to the bending section 120, prevents misalignment of the distal tip 110 and the bending section 120, and helps improve overall quality of the endoscope.

Specifically, two first engaging posts 114 are provided, two second engaging posts 115 are provided. The two engaging posts 114 are arranged adjacent to each other, and the two second engaging posts 115 are arranged adjacent to each other. Correspondingly, two first engaging holes 123 are provided, two second engaging holes 124 are provided. The two engaging holes 123 are arranged adjacent to each other, and the two second engaging holes 124 are arranged adjacent to each other. In this way, it is beneficial to prevent the distal tip 110 from being reversed during mounting, thereby improving convenience and efficiency of mounting the distal tip 110.

In an embodiment, referring to FIGS. 1, 2, and 3, the cross-sectional profile of the first engaging post 114 is in a waist shape extending in the axial direction of the distal tip 110, and the opening shape of the first engaging hole 123 is in a waist shape extending in the axial direction of the bending section 120. The cross-sectional profile of the second engaging post 115 is circular, and the opening shape of the second engaging hole 124 is circular. Although this embodiment provides only an option for the shapes of the engaging posts 112 and the engaging holes 122, but which are not limited thereto.

In order to understand and illustrate the axial direction of the distal tip 110, as show in FIG. 3, the axial direction of the tip 110 is a direction indicated by any arrow on a straight line Si in FIG. 3. In addition, the bending section 120 is coaxially connected to the distal tip 110.

Specifically, referring to FIGS. 1, 2, and 3, the distal tip 110 is made of polycarbonate (PC), and the bending section 120 is made of polymethanol (POM). In this way, materials for manufacture are at a low cost and are non-toxic and harmless, which, on the one hand, is beneficial to reduce the manufacturing cost and improve economic efficiency of the product, and on the other hand, is beneficial to prevent secondary injuries such as allergies, and improve quality of use of the endoscope. Although this embodiment provides only an option for the materials for the distal tip 110 and the bending section 120, but which are not limited thereto.

In an embodiment, referring to FIGS. 2 and 3, the distal tip 110 is provided with a first annular connecting portion 116. The first annular connecting portion 116 is connected to the distal tip 110 and extends in the axial direction of the distal tip 110. The bending section 120 is provided with a second annular connecting portion 125. The second annular connecting portion 125 is connected to the bending section 120 and extends in the axial direction of the bending section 120. The first engaging portions 111 are located in the circumferential direction of the first annular connecting portion 116, the second engaging portions 121 are located in the circumferential direction of the second annular connecting portion 125. An outer diameter of the first annular connecting portion 116 is less than an inner diameter of the second annular connecting portion 125. The second annular connecting portion 125 is sleeved outside the first annular connecting portion 116. The distal tip 110 is engaged with the second annular connecting portion 125, and an outer wall of the distal tip 110 smoothly transitions to an outer wall of the second annular connecting portion 125. In this way, after the bending section 120 is connected to the distal tip 110, smooth transition between outer walls thereof is beneficial to improve structural flatness, and thus prevent damages to organ tissues when entering human organs, thereby improving overall quality of use of the endoscope.

Further, a sealant is applied between an outer wall of the first annular connecting portion 116 and the inner wall of the second annular connecting portion 125. In this way, the first annular connecting portion 116 and the second annular connecting portion 125 are connected by snap-fitting and bonding, which helps to further improve the stability of the connection between the distal tip 110 and the bending section 120, thereby preventing the distal tip 110 from falling off the bending section 120, ensuring sealing performance, and improving the overall quality of the endoscope.

Alternatively, in another embodiment, the distal tip 110 is provided with a first annular connecting portion 116. The first annular connecting portion 116 is connected to the distal tip 110 and extends in the axial direction of the distal tip 110. The bending section 120 is provided with a second annular connecting portion 125. The second annular connecting portion 125 is connected to the bending section 120 and extends in the axial direction of the bending section 120. The first engaging portions 111 are located in the circumferential direction of the first annular connecting portion 116, the second engaging portions 121 are located in the circumferential direction of the second annular connecting portion 125. An outer diameter of the second annular connecting portion 125 is less than an inner diameter of the first annular connecting portion 116. The first annular connecting portion 116 is sleeved outside the second annular connecting portion 125. The bending section 120 is engaged with the first annular connecting portion 116. An outer wall of the bending section 120 smoothly transitions to an outer wall of the first annular connecting portion 116. In this way, after the bending section 120 is connected to the distal tip 110, smooth transition between outer walls thereof is beneficial to improve structural flatness, and thus prevent damages to organ tissues when entering human organs, thereby improving overall quality of use of the endoscope.

Further, a sealant is applied between an outer wall of the second annular connecting portion 125 and an inner wall of the first annular connecting portion 116. In this way, the first annular connecting portion 116 and the second annular connecting portion 125 are connected by snap-fitting and bonding, which helps to further improve the stability of the connection between the distal tip 110 and the bending section 120, thereby preventing the distal tip 110 from falling off the bending section 120, ensuring sealing performance, and improving the overall quality of the endoscope.

In an embodiment, an endoscope (not shown) includes the connecting structure for a distal tip and a bending section 100 according to the above embodiments.

In the above endoscope, during mounting, the first engaging portions 111 on the distal tip 110 are snap-fitted with the second engaging portions 121 on the bending section 120, such that the distal tip 110 is connected to the bending section 120. The snap-fit connection facilitates mounting, is suitable for the connection between the bending section 120 made of plastic materials and the distal tip 110made of plastic materials, and is beneficial for improving the operating convenience of the connection between the bending section 120 and the distal tip 110. In addition, the first engaging portions 111 are provided in the circumferential direction of the distal tip 110, and the second engaging portions 121 are provided in the circumferential direction of the bending section 120, and the connection between the first engaging portions and the second engaging portions can improve the stability of the connection between the distal tip 110 and the bending section 120, thereby preventing the distal tip 110 from falling off the bending section 120, improving the reliability of the endoscope, and reducing the probability of medical accidents.

The technical features in the above embodiments may be randomly combined. For concise description, not all possible combinations of the technical features in the above embodiments are described. However, all the combinations of the technical features are to be considered as falling within the scope described in this specification provided that they do not conflict with each other.

The above embodiments only describe several implementations of the present application, and their description is specific and detailed, but cannot therefore be understood as a limitation on the patent scope of the present application. It should be noted that those of ordinary skill in the art may further make variants and improvements without departing from the conception of the present application, and these all fall within the protection scope of the present application. Therefore, the patent protection scope of the present application should be subject to the appended claims.

## Claims

1. A connecting structure for a distal tip and a bending section, comprising:
a distal tip provided with first engaging portions, the first engaging portions being located in a circumferential direction of the distal tip; and
a bending section coupled to the distal tip, the bending section being provided with second engaging portions, the second engaging portions being located in a circumferential direction of the bending section, and the first engaging portions being snap-fitted with the second engaging portions, such that the distal tip is connected to the bending section.

2. The connecting structure for a distal tip and a bending section according to claim 1, wherein at least two first engaging portions and at least two second engaging portions are provided; the at least two first engaging portions being arranged at intervals in the circumferential direction of the distal tip; the at least two second engaging portions being arranged at intervals in the circumferential direction of the bending section; and the at least two first engaging portions and the at least two second engaging portions being arranged in one-to-one correspondence.

3. The connecting structure for a distal tip and a bending section according to claim 2, wherein the first engaging portions are formed as engaging posts, the second engaging portions are formed as engaging holes; and cross-sectional profiles of the engaging posts are adapted to opening shapes of the engaging holes respectively; or
the first engaging portions are formed as engaging holes, the second engaging portions are formed as engaging posts; and opening shapes of the engaging holes are adapted to cross-sectional profiles of the engaging posts respectively.

4. The connecting structure for a distal tip and a bending section according to claim 3, wherein the engaging posts are provided with introducing inclined surfaces respectively; the introducing inclined surfaces being configured to guide the engaging posts into the engaging holes respectively.

5. The connecting structure for a distal tip and a bending section according to claim 3, wherein the at least two engaging posts comprise at least a first engaging post and a second engaging post; the at least two engaging holes comprise at least a first engaging hole and a second engaging hole; the first engaging post being snap-fitted with the first engaging hole; and the second engaging post being snap-fitted with the second engaging hole; a cross-sectional profile of the first engaging post is different from that of the second engaging post; and an opening shape of the first engaging hole is different from that of the second engaging hole.

6. The connecting structure for a distal tip and a bending section according to claim 5, wherein the cross-sectional profile of the first engaging post is in a waist shape extending in an axial direction of the distal tip; and the opening shape of the first engaging hole is in a waist shape extending in an axial direction of the bending section; and the cross-sectional profile of the second engaging post is circular, and the opening shape of the second engaging hole is circular.

7. The connecting structure for a distal tip and a bending section according to any one of claims 1 to 6, wherein the distal tip is provided with a first annular connecting portion; the first annular connecting portion being connected to the distal tip and extending in an axial direction of the distal tip; the bending section is provided with a second annular connecting portion; the second annular connecting portion being connected to the bending section and extending in an axial direction of the bending section; the first engaging portions are located in a circumferential direction of the first annular connecting portion; the second engaging portions are located in a circumferential direction of the second annular connecting portion; an outer diameter of the first annular connecting portion is less than an inner diameter of the second annular connecting portion; the second annular connecting portion being sleeved outside the first annular connecting portion; the distal tip is engaged with the second annular connecting portion; and an outer wall of the distal tip smoothly transitions to an outer wall of the second annular connecting portion.

8. The connecting structure for a distal tip and a bending section according to claim 7, wherein a sealant is applied between an outer wall of the first annular connecting portion and an inner wall of the second annular connecting portion.

9. The connecting structure for a distal tip and a bending section according to any one of claims 1 to 6, wherein the distal tip is provided with a first annular connecting portion; the first annular connecting portion being connected to the distal tip and extending in an axial direction of the distal tip; the bending section is provided with a second annular connecting portion; the second annular connecting portion being connected to the bending section and extending in an axial direction of the bending section; the first engaging portions are located in a circumferential direction of the first annular connecting portion; the second engaging portions are located in a circumferential direction of the second annular connecting portion; an outer diameter of the second annular connecting portion is less than an inner diameter of the first annular connecting portion; the first annular connecting portion being sleeved outside the second annular connecting portion; the bending section is engaged with the first annular connecting portion; and an outer wall of the bending section smoothly transitions to an outer wall of the first annular connecting portion.

10. The connecting structure for a distal tip and a bending section according to claim 9, wherein a sealant is applied between an outer wall of the second annular connecting portion and an inner wall of the first annular connecting portion.

11. An endoscope, wherein the endoscope comprises the connecting structure for a distal tip and a bending section according to any one of claims 1 to 10.
